Europäisches Patentamt

**European Patent Office**

**Office européen des brevets**

(11) Publication number: **0 383 363**
**A2**

(12) **EUROPEAN PATENT APPLICATION**

(21) Application number: **90106079.8**

(51) Int. Cl.5: **A61K 37/02**, //C07K3/02

(22) Date of filing: **08.11.85**

This application was filed on 29 - 03 - 1990 as a divisional application to the application mentioned under INID code 60.

(30) Priority: **29.11.84 US 676471**
**10.10.85 US 786206**

(43) Date of publication of application:
**22.08.90 Bulletin 90/34**

(60) Publication number of the earlier application in accordance with Art.76 EPC: **0 202 298**

(84) Designated Contracting States:
**AT BE CH DE FR GB IT LI LU NL SE**

(71) Applicant: **CURATECH, INC.**
**1201 Marquette Avenue, Suite 400**
**Minneapolis, MN 55403(US)**

(72) Inventor: **The designation of the inventor has not yet been filed**

(74) Representative: **Strehl, Schübel-Hopf, Groening**
**Maximilianstrasse 54 Postfach 22 14 55**
**D-8000 München 22(DE)**

(54) **Use of wound healing agents.**

(57) A composition comprising a first substance which causes direct growth of capillary endothelium and a second substance which is mitogenic for cells selected from the group consisting of fibroblast cells, capillary endothelial cells and epithelial cells is useful for preparing a medicament to be applied to wounds for the purpose of forming granulation tissue in the wounds or may be used for cosmetic purposes.

EP 0 383 363 A2

## USE OF WOUND HEALING AGENTS

### Field of the Invention

This invention relates to wound healing agents, specifically angiogenic and growth factors, their production from blood and their use to prepare a pharmaceutical composition which facilitates the healing of wounds and a composition for cosmetic purposes.

### Background of the Invention

Angiogenesis, which is the proliferation and directed growth of capillary endothelium, along with fibroplasia and collagen synthesis are integral components of a host's response to wounding. The activation of platelets and the clotting cascade are among the first reactions to injury.

Platelets activated by thrombin release a mitogen, or growth factor, for fibroblasts and smooth muscle cells and stimulate increased collagen synthesis by smooth muscle cells in vitro. The mitogen, (platelet-derived growth factor, hereinafter PDGF) is composed of two polypeptides. An article describing PDGF was published in 1982 by G.A. Grotendorst, T. Chang, H.E.J. Seppa, H.K. Kleinman and G.A. Martin in the Journal of Cellular Physiology entitled "Platelet-Derived Growth Factor is a Chemoattractant for Vascular Smooth Muscle Cells", Vol. 113, pp. 261-266. The article is incorporated herein by reference.

A non-mitogenic substance, called angiogenic factor, is also produced by thrombin activated platelets and stimulates capillary growth. Various angiogenesis factors are known including tumor, retinal and wound fluid angiogenesis factors. It is unknown whether all angiogenesis factors share a common mechanism of action upon capillary endothelial cells.

Angiogenesis factors were isolated and described by M.S. Banda, D.R. Knighton, T.K. Hunt and Z. Werb in Proc. Nat'l. Acad. Sci. U.S.A. (7773 - 7777, Dec. 1982), in an article entitled "Isolation of a nonmitogenic angiogenesis factor from wound fluid", the disclosure of which is incorporated herein by reference.

Angiogenesis and platelet derived growth factors are described by D.R. Knighton, T.K. Hunt, K.K. Thakral and W.H. Goodson III, in "Role of Platelets and Fibrin in the Healing Sequence," Annals of Surgery 196: 379-388 (1982), the disclosure of which is incorporated by reference. In this article, the successful treatment of a non-healing wound in a patient is described in which a single, ten-unit platelet transfusion was given. The wound healed in three weeks.

A recent study has indicated that when the body's normal healing process works, it is only at about a 50% effectiveness level.

A human angiogenic factor is produced from human foreskin fibroblasts in United States Patent 4,273,871 to Tolbert et al. A publically available foreskin fibroblast cell line is utilized to produce an angiogenic factor.

In United States Patent 4,479,896 to Antoniades the disclosure of which is incorporated herein by reference, platelet-derived growth factors are characterized and extracted for study by gel electrophoresis means.

### Brief Summary of the Invention

The present invention relates to the use of a composition, comprising a first substance which causes direct growth of capillary endothelium and a second substance which is mitogenic for cells selected from the group consisting of fibroblast cells, capillary endothelial cells and epithelial cells for preparing a medicament for the application to wounds for the purpose of forming granulation tissue in the wounds.

The invention furthermore relates to the use of a composition which comprises platelet derived growth factor for the preparation of a medicament for the topical application to a wound for the purpose of forming granulation tissue in the wound.

Furthermore, the present invention is directed to a method of cosmetic treatment of tissue comprising applying a composition topically onto such tissue said composition comprising a first substance which causes directed growth of capillary endothelium.

According to another embodiment, this invention relates to the use of a composition for the application to tissue in order to form granulation tissue and/or capillaries and/or epithelial tissue, said composition comprising

(a) material released from mammalian platelets, and

(b) a pharmaceutically acceptable carrier or diluent therefor,

wherein said composition is substantially free of (i) blood or plasma contaminants and (ii) platelet ghosts or other material found in said platelets but not released by said platelets, for cosmetic purposes.

Thrombin activated platelets have the capacity to stimulate angiogenesis, increased collagen synthesis and cell division and growth. It has been found that samples of whole blood may be utilized to prepare a platelet-enriched plasma, which when activated by thrombin, contains angiogenic and growth factors which may be used to speed the healing process of wounds.

Blood is stabilized and centrifuged to obtain a platelet-rich plasma. The blood is stabilized by mixing with citrate-phosphate-dextrose in a ratio of 1:5 (20% solution). The platelet-rich plasma (hereinafter PRP) is preferably centrifuged again until a high concentration of platelets is obtained. The platelets are then placed in a platelet buffer. The concentration of platelets should be at least 1,000,000 platelets per milliliter. Preferably, the concentration should be on the order of 1,000,000,000 platelets per milliliter.

Thrombin is added to the PRP in order to activate the platelets. Preferably, about 1 to about 10 units of thrombin are utilized per milliliter of PRP. The thrombin-activated platelets release platelet derived growth factors (hereinafter PDGF) and platelet derived angiogenesis factors (hereinafter PDAF). The platlets and thrombin are allowed to incubate at room temperature for about 5 to 10 minutes.

The activated PRP containing PDGF and PDAF is preferably added to a biologically compatible macromolecular substance which acts as a carrier. First the platlets are centrifuged at about 950 x g and the platelet free supernatant is mixed with the carrier. Preferably, a microcrystalline collagen such as Avitene® brand collagen as sold by FMC Corp., Avicel Dept., Marcus Hook, PA 19061 is utilized as the biologically compatible carrier. Micro-crystalline collagens are biologically compatible in the body. Enough carrier is added to soak up all the platelet rich plasma that is obtained from the blood. For examples, a 40ml blood sample would typically require about 25ml of carrier after enrichment. The paste so obtained is preferably stored on ice or in the refrigerator.

The pharmaceutical preparations for use as a wound dressing sold by Pharmacia Fine Chemicals, Inc. of Piscataway, New Jersey under the trademark Debrisan is a suitable carrier.

The activated PRP within the carrier may then be applied to a wound. The highly enriched and active PDGF and PDAF therewithin assists in healing by proliferating and directing the growth of capillary endothelium, doubling the rate of collagen synthesis and by producing leukocyte chemotaxis. Mitogenic activity results in cellular division and growth to replace the lost tissue.

Daily application of the activated PRP to wounds stimulates and bolsters the healing sequence. The amount of PRP processed from 40ml of blood is enough to produce applications for seven days. The material is placed over the entire wound at a relatively uniform thickness, approximately two millimeters thick. Granulation, contraction and epithelization may be initiated through the use of activated PRP where the body's own repair signals are inadequate to stimulate good healing.

Whenever thrombin is used herein, it is referring to thrombin as a biologic release agent for platelet release. Other biologic release agents known in the art, including collagen, ADP and serotonin, may be utilized instead of or in addition to thrombin to activate the platelets, although thrombin is preferred.

Detailed Description of the Invention

Blood obtained from the individual to be treated with the wound healing factors of the invention is stabilized in siliconized tubes containing acid-citrate dextrose (0.15M citrate, 2% glucose, pH 4.2) (hereinafter CPD) and is centrifuged in order to separate out the platelet-rich plasma therefrom. Forty to sixth milliliters of blood combined with 4-6ml of CPD is then centrifuged at about 135 x 4 g for 20 minutes at about 4°C to obtain platelet-rich plasma. The platelet rich plasma is removed and placed into another sterile, 50ml tube. A platelet count is then taken. The CDP is utilized to prevent activation of the clotting sequence by contact of the blood with the plastic in the syringe. The CPD is present in the syringe while the blood is withdrawn from the patient. The blood is continuously mixed with the CPD to prevent coagulation. The platelet-rich plasma in the tube is then centrifuged at 750 x g for 10 minutes at 4°C.

The platelet-free plasma is removed and discarded. The platelet pellet is resuspended in a quantity of platelet buffer to produce a final ml. A lower concentration of about a million platelets per ml is useful, but is less preferred. The platelet buffer utilized contains .05 M HEPES (N-2-hydroxyethylpiperazine-n-2-ethanesulfonic acid), 0.03 M glucose, 0.004 M KCl, 0.1 M NaCl and about 0.35% human serum albumin adjusted to a pH of about 6.5. A sample is frozen at about -20°C for later testing of mitogenic activity. Another sample is streaked onto blood agar as a sterility test.

The platelet-rich plasma is the only blood fraction utilized in the processes and compositions of the invention. The PRP is then activated with purified thrombin at a rate of about 1 to about 10 units of thrombin per milliliter of PRP. Preferably, about 1 unit of thrombin per ml of platelet-rich plasma is utilized. The activity of the thrombin coagulates the fibrinogen and activates platelets causing them to release alpha granules containing platelet-derived growth factor and platelet-derived angio-genesis factor. The thrombin used was Thrombinar" brand from Armour Pharmaceutical Co. of Kankakee, Il-linois. The platelets and thrombin are allowed to incubate at room temperature for about 5-10 minutes.

The PRP is then subjected to a removal of platelets and fibrin by centrifugation. The resulting supernatant contains both PDAF and PDGF after centrifuging at 950 x g for about 5 minutes at 4° C. The pellet is discarded since the PDAF & PDGF have been extracted into the supernatant. PDGF has been isolated and characterized. It is a protein of 30,000 molecular weight which breaks down into two molecular weight species of 15,000 and 14,000 molecular weight.

In order to apply the PDAF and PDGF in the platelet-free supernatant thus obtained to a wound, it is desirable to utilize a carrier substance which is biologically compatible and acts as a temporary "depot". A macromolecular substance such as microcrystalline collagen provides a suitable carrier. An especially preferred carrier is Avitene® brand microcrystalline collagen from FMC Corp., Avicel Dept., Marcus Hook, PA 19061. The resultant composition is thicker and will tend to remain in position in contact with the wound. Debrisan™ brand wound dressing which contains Sepharose™ brand beads, trademarks of Pharmacia Fine Chemicals, Inc. of Piscataway, New Jersey, may be utilized as an alternative carrier. Preferably, about 8-10ml of supernatant per gram of carrier is used to produce a paste.

Application of the wound treating composition is by physically applying the material over an into the wound as in applying a medicated salve. Treatments should be repeated on a daily basis as long as the wound remains open. A preferred treatment is to apply an approximately one mm thick dressing of the platelet factor/carrier complex to the wound in the morning. It is then dressed with a sterile, dry dressing. In the evening, the dressing is removed and the substance is removed by washing with sterile saline.

Although the clinical testing involving the wound treating compositions of the invention have been directed to wounds on the body exterior, the compositions may treat internal wounds as well. Sutures may be impregnated with the wound treating compositions to speed internal healing. The wound treating compositions may also be used in conjunction with biodegradable dressings, as a coating over implantable devices and biodegradable devices utilized in surgical procedures. Generally, any foreign body to be inserted into a patient may be coated with the composition to speed the healing process. Alternatively, the composition may be applied over the damaged tissue directly.

Initial clinical trials have been performed on eight patients, all with nonhealing wounds from periods of one to five years. All patients had maximal standardized care in attempts to heal the wounds. That therapy had failed. In all cases, administration of platelet-derived factors initiated a healing response as evidenced by granulation tissue formation (granulation tissue contains fibroblasts, endothelial cells and collagen). The wounds closed by contraction and epithelialization or by skin grafting. Stimulation of healing and eventual repair occurred in all applications.

While it is preferred to prepare activated PRP for wound treatment purposes directly from the injured animal's own blood, the advantages of the invention may be achieved by using blood or outdated platelets from animals of the same species. Utilization of blood from the injured individual to be treated is especially preferred since it avoids exposure to possible hepatitis or other contaminants from banked blood. The use of a patient's own blood would also eliminate any possible allergic reactions. A consistent source of the material may be obtained from washed, outdated human platelets. The substances may also be utilized in veterinary applications by utilizing platelets derived from the animal itself or another animal within the same species.

Example I

A patient having an open wound on the left foot following debridement of dead tissue and transmetatarsal amputation was started on PDGF and PDAF obtained as described above from his own blood. After the treatment protocol, the wound was filled with new granulation tissue. A subsequent debridement showed completely covered metatarsal bones and contracture of the sizable wound.

Example II

A patient underwent amputation of his right great toe and was treated with standard therapy for three weeks without any granulation tissue accumulating within the wound. He was then started on the platelet factor therapy of the invention. After three weeks of treatment, the wound contracted approximately 30-40% and was healing rapidly.

Example III

A patient having two large wounds on the medial and lateral aspect of his transmatatarsal amputation stump had been treated for four months without healing using conventional therapy. Within two weeks of treatment with PDAF and PDGF as described above, the wound had cleared of an apparent infection and started producing granulation tissue.

Thirty-eight nonhealing ulcers from 28 diabetic patients were treated with the PRP paste. The average duration of the ulcers before treatment was 6-1/2 years. A paste prepared from PRP at a concentration of about $10^9$ platelets/ml was combined with Avitene brand collagen. The patients applied the PDGF and PDAF containing paste daily for 19 hour periods for an average of 8 weeks. Each day, the wounds were debrided of dead tissue. All of the wounds produced granulation tissue and closed an average of 83% when compared to starting wound area. Ninety-five percent of the ulcers were successfully treated resulting in either total wound epithlialization or successful skin grafting. Only two of these nonhealing wounds did not heal. The healed ulcers remain closed with no evidence of hypertrophic scar formation or neoplastic formation.

In considering this invention, it should be remembered that the disclosure is illustrative only, and that the scope of the invention should be determined by the appended claims.

## Claims

1. Use of a composition comprising a first substance which causes direct growth of capillary endothelium and a second substance which is mitogenic for cells selected from the group consisting of fibroblast cells, capillary endothelial cells and epithelial cells for preparing a medicament for the application to wounds for the purpose of forming granulation tissue in the wounds.

2. Use according to claim 1, wherein said first substance is chemotactic for capillary endothelial cells.

3. Use according to claim 2, wherein said first substance is platelet-derived angiogenesis factor (PDAF).

4. Use according to any of claims 1 to 3, wherein said second substance is platelet-derived growth factor (PDGF).

5. Use of a composition comprising a substance which is both chemotactic and non-mitogenic for capillary endothelial cells, for preparing a medicament for the topical application to tissue for the purpose of causing directed growth of capillary endothelium.

6. Use according to claim 5, wherein said composition further comprises a second substance which is mitogenic for cells selected from the group consisting of fibroblast cells, capillary endothelial cells and epithelial cells.

7. Use according to claim 6, wherein the second substance is platelet derived growth factor.

8. Use according to claim 6, for preparing a medicament for the topical application to a wound for the purpose of causing substantial formation of granulation tissue in said wound.

9. Use according to any of claims 5 to 8, wherein the tissue is mammalian tissue.

10. Use according to claim 9, wherein said tissue is human tissue.

11. Use of a composition comprising platelet derived growth factor for the preparation of a medicament for the topical application to a wound for the purpose of forming granulation tissue in the wound.

12. Use according to claim 11, wherein said platelet derived growth factor is mammalian platelet derived growth factor.

13. Use according to claim 12, wherein said platelet-derived growth factor is a growth factor derived from mammalian platelets selected from the group consisting of bovine platelets, porcine platelets, murine platelets, rabbit platelets, human platelets, primate platelets and equine platelets.

14. Use according to claim 13, wherein said platelet-derived growth factor is human platelet-derived growth factor.

15. A method of cosmetic treatment of tissue comprising applying a composition topically onto such tissue said composition comprising a first substance which causes directed growth of capillary endothelium.

16. A method according to claim 15 wherein the composition topically applied to the tissue comprises a substance which is both chemotactic and non-mitogenic for capillary endothelial cells.

17. A method according to any of claims 15 or 16, wherein said composition further comprises a second substance which is mitogenic for cells selected from the group consisting of fibroblast cells, capillary endothelial and epithelial cells.

18. A method according to any of claims 15 to 17, wherein the composition comprises a material released from platelets.

19. The method according to claims 15 to 18, wherein said platelets are isolated from blood prior to release of said material.

20. A method according to claim 19, wherein said platelets are mammalian platelets.

21. A method according to claim 20, wherein said platelets are mammalian platelets selected from the group consisting of bovine platelets, porcine platelets, murine platelets, rabbit platelets, human platelets, primate platelets and equine platelets.

22. A method according to claim 21, wherein said platelets are human platelets.

23. A method to any of claims 15 to 22, wherein the composition applied to tissue comprises

(a) material released from mammalian platelets, and

(b) a pharmaceutically acceptable carrier or diluent therefor,

wherein said composition is substantially free of (i) blood or plasma contaminants and (ii) platelet ghosts or other material found in said platelets but not released by said platelets.

24. A method according to any of claims 15 to 23, wherein said material released from said platelets comprises a combination of platelet-derived angiogenesis factor and platelet-derived growth factor.

25. Use of a composition for the application to tissue in order to form granulation tissue and/or capillaries and/or epithelial tissue, said composition comprising

(a) material released from mammalian platelets, and

(b) a pharmaceutically acceptable carrier or diluent therefor,

wherein said composition is substantially free of (i) blood or plasma contaminants and (ii) platelet ghosts or other material found in said platelets but not released by said platelets, for cosmetic purposes.

26. Use according to claim 25, wherein the concentration, per milliliter of said composition, of said material released from said mammalian platelets is within the range of concentration equivalent to the amount of material released from about $10^6$ to about $10^9$ platelets.

27. Use according claim 25, wherein said material released from said mammalian platelets comprises a combination of platelet derived angiogenesis factor and platelet derived growth factor.

28. Use according to any of the claims 25 to 27, wherein said material released from said mammalian platelets is released from platelets selected from the group consisting of bovine platelets, porcine platelets, murine platelets, rabbit platelets, human platelets, primate platelets and equine platelets.

29. Use according to claim 28, wherein said material released from said mammalian platelets is released from human platelets.